# EUROPEAN PATENT APPLICATION

(11) **EP 2 657 865 A1**
(43) Date of publication of application: **30.10.2013**
(21) Application number: 12002907.9
(22) Date of filing: 25.04.2012
(51) Int. Cl.: G06F 19/00

(54) **Method of creating exercise routes for a user**

(71) Applicant: Mitac International Corporation, Tao-Yuan Hsien (TW)
(72) Inventor: Van Hende, Iwan, Tao-Yuan Hsien (TW)
(74) Representative: Hoefer & Partner

(57) **Abstract**

A method of creating a customizable exercise route for a user of a personal navigation device (10) includes receiving a selection of terrain type or difficulty level from the user, receiving a selection of a length of an exercise route to be generated, generating an exercise route for the user according to a current position of the personal navigation device (10), the selected terrain type or difficulty level, and the selected length of the exercise route, and providing routing instructions for the user to follow the generated exercise route.

## Description

The present invention relates to a method of creating customized exercise routes for a user of a personal navigation device based on parameters input by the user.

Currently, personal navigation devices used for outdoor activities such as hiking, running, or cycling are limited in their options presented to users. Route planning with conventional personal navigation devices is performed by the user entering a starting point and an ending point, with the personal navigation devices creating a planned route for the user based on these criteria. However, there are very few options available to the user for creating varied exercise routes that satisfy additional user requirements. When a user wants to drive from point A to point B as quickly as possible, the personal navigation device has fewer user requirements to satisfy, and usually aims to provide the user with the fastest route possible.

However, when it comes to leisure activities or exercise routes where creating the fastest route is not necessarily the most important factor, users demand the ability to create more flexible and diverse routes. Unfortunately, when creating a route between point A and point B, conventional personal navigation devices always provide the same route and do not offer any alternatives. Sports-based personal navigation devices also do not always provide any personalized routes based on specific user requirements or existing training programs followed by the user.

This in mind, the present invention aims at providing a corresponding method of creating exercise routes for a user.

This is achieved by a method of creating a customizable exercise route for a user of a personal navigation device according to claim 1. The dependent claims pertain to corresponding further developments and improvements.

As will be seen more clearly from the detailed description following below, the claimed method provides a large assortment of options to be chosen by the user in order to create a customized exercise route. In this way, the user has considerably more control over what type of route will be selected while still retaining the convenience of the personal navigation device helping plan the route, the user can exercise on a greater variety of routes, and the user can continue training on level-appropriate exercise routes. Moreover, having a greater variety of training options makes it more likely that the user will find and continue with a training program that is enjoyable and challenging to the user.

In the following, the invention is further illustrated by way of example, taking reference to the accompanying drawings. Thereof
Fig. 1 is a block diagram of a personal navigation device according to the present invention.
Fig. 2 is a flowchart illustrating a method of creating customizable exercise routes using the personal navigation device according to the present invention.
Fig. 3 shows terrain preferences that may be selected by the user of the personal navigation device.
Fig. 4 shows detailed terrain preferences that may be selected by the user of the personal navigation device.
Fig. 5 shows route length preferences that may be selected by the user of the personal navigation device.
Fig. 6 is a screen showing input route parameter preferences selectable by the user.
Figs. 7-9 are block diagrams of personal navigation devices according to other embodiments of the present invention.
Fig. 10 is a screen showing settings for creating suggested exercise routes with the surprise me function using a combination of exercise time and average speed at which the user will move while exercising.
Fig. 11 is a screen showing settings for creating suggested exercise routes with the surprise me function using a distance that the user will travel while exercising.
Fig. 12 is a screen showing a map containing three suggested exercise routes displayed on the map.
Fig. 13 depicts a workout screen for allowing the user to enter parameters for a desired workout using a combination of distance that the user will travel while exercising and average speed at which the user will move while exercising.
Fig. 14 depicts a workout screen for allowing the user to enter parameters for a desired workout using a combination of exercise time and average speed at which the user will move while exercising.

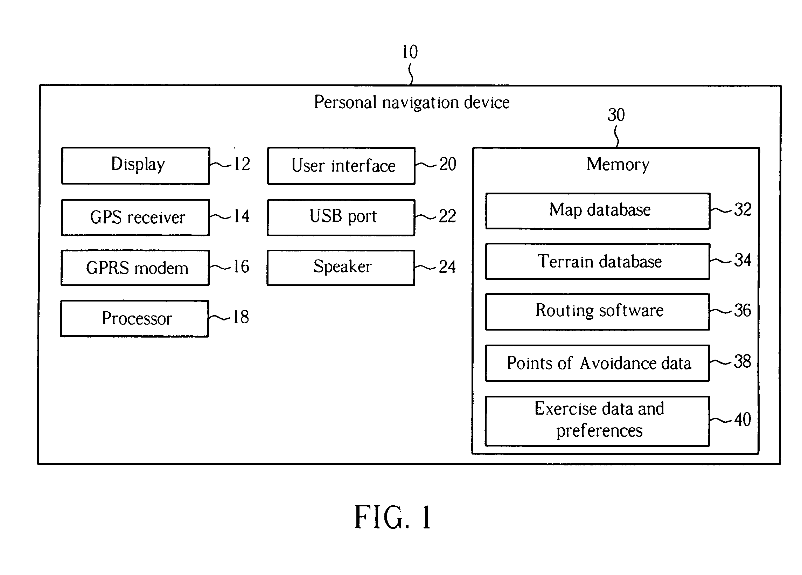

Please refer to Fig. 1. Fig. 1 is a block diagram of a personal navigation device 10 according to the present invention. The personal navigation device 10 contains a display 12 which can be a touch sensitive display, a GPS receiver 14 for receiving the current coordinates of the personal navigation device 10, a General Packet Radio Service (GPRS) modem 16 for providing internet access and data connectivity, a processor 18 for controlling operation of the personal navigation device 10, a user interface 20, a Universal Serial Bus (USB) port 22 for allowing data to be exchanged with a computer, a speaker 24, and memory 30. Instead of the speaker 24, a buzzer can also be used to provide audio feedback to the user. The memory 30 is used to store a map database 32 containing map data and points of interest, a terrain database 34 containing terrain information of at least some of the areas covered by the map database 32, and routing software 36. The memory 30 also stores points of avoidance data 38 which contains areas that users may wish to avoid based on historical safety data of that area. The user of the personal navigation device 10 can download updated points of avoidance data 38 through the USB port 22 or through the GPRS modem 16. User data such as exercise data and preferences 40 is also stored in the memory 30 in order to have a record of what training level the user is at, what exercise routes the user prefers, and what length of exercise routes the user prefers. The personal navigation device 10 can also be provided with a camera for allowing the user to take geotagged photographs and to share them with others.

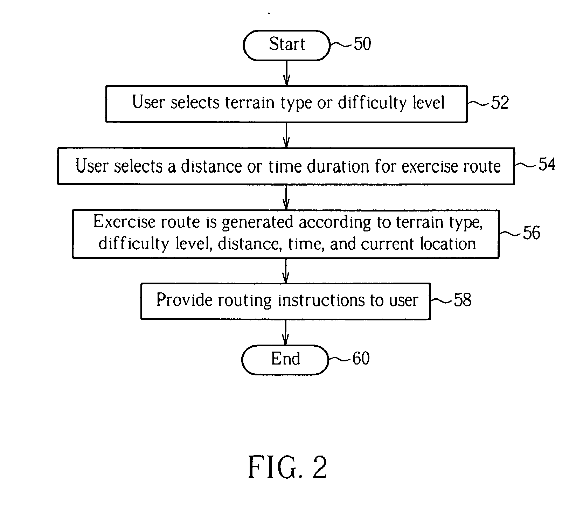

Please refer to Fig. 2. Fig. 2 is a flowchart illustrating a method of creating customizable exercise routes using the personal navigation device 10 according to the present invention. Steps contained in the flowchart will be explained below.

### Step 50: Start.

Step 52: The user selects a terrain type or difficulty level for the exercise route. The user may specify the user's mode of transportation, such as walking, off-road cycling, road cycling, wheelchair, electric bike, and motorbike for selecting a route that is suitable for this activity. Other terrain options can also be specified, such as routes that have nearby parking, routes with hilly terrain, routes with flat terrain, routes in urban areas, routes in a green belt area or a natural area, routes that have a significant number of stairs, or routes along a beach. The user may also ask for routes to be generated according to a difficulty level of the route, according to a difficulty scale of 1 to 5 or easy, medium, and difficult.

Step 54: The user indicates a length of the route to be generated. The length can be indicated in distance, or can be indicated in the amount of time that the user would like to exercise. If the user specifies a distance for the exercise route, the routing software 36 can search for an exercise route having a total distance approximately equal to the desired distance. If the user specifies an exercise time period, the routing software 36 can then use estimated speed information that may be based on the user's training history for the potential exercise routes in order to find an exercise route that would allow the user to exercise for the desired amount of time. The user may ask for a one-way route to be generated, which guides the user from point A to point B, or for a loop route to be generated which guides the user back to the original starting point at the end of the exercise route.

Step 56: The routing software 36 generates an exercise route according to the user's preferred terrain type or difficulty level and route length. The current position of the personal navigation device 10 given by the GPS receiver 14 is also consulted for locating an exercise route nearby the user's current position. Points of interest selected by the user can also be used for generating the exercise route.

Step 58: The routing software 36 provides routing instructions to the user. Visual instructions can be given to the user through the display 12. Audio instructions can also be given through the speaker 24, a Bluetooth headset, or through an earphone jack. When users are cycling or walking, they may prefer to hear audio instructions instead of having to stop and glance down at the display 12, so preferably both audio and visual instructions are given to the user.

Step 60: End.

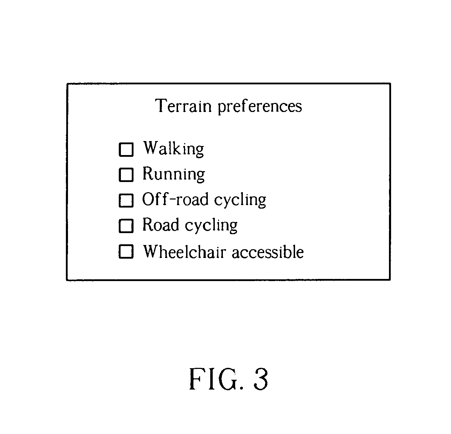

Please refer to Fig. 3. Fig. 3 shows terrain preferences that may be selected by the user of the personal navigation device 10. These are broad choices indicating the user' s mode of transportation that can later be refined depending on the user's selection. The choices include walking routes, running routes, off-road cycling routes, road cycling routes, and wheelchair accessible routes. Other potential terrain possibilities include paved roads or paths, unpaved roads or paths, urban areas, rural areas, avoiding urban areas, and avoiding rural areas.

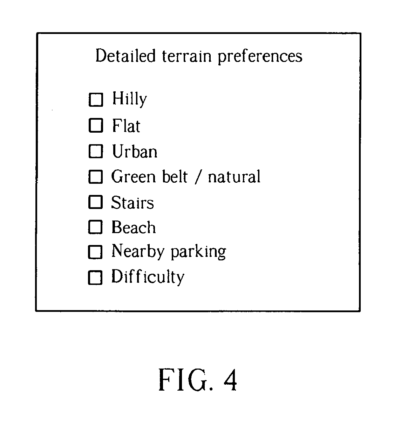

After an initial choice is made, the user can be given further options. Suppose that the user selected the choice for "Walking" in Fig. 3. More detailed terrain options could then be presented to the user for allowing the user to select what kind of walking route is preferred. Please refer to Fig. 4. Fig. 4 shows detailed terrain preferences that may be selected by the user of the personal navigation device 10. These choices include routes with hilly terrain, routes with flat terrain, routes in urban areas, routes in a green belt area or a natural area, routes that have a significant number of stairs (measured as a total number of stairs along the route or as a number of stairs per unit distance), routes along a beach, or routes that have nearby parking. The user may also ask for routes to be generated according to a difficulty level of the route, such as on a difficulty scale of 1 to 5.

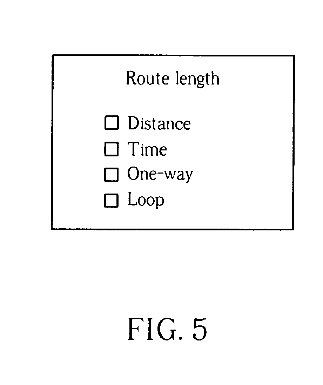

After the user has selected a terrain type or difficulty level, the user is given the chance to specify the length of the route and whether the route is a one-way route or a loop route. Please refer to Fig. 5. Fig. 5 shows route length preferences that may be selected by the user of the personal navigation device 10. As stated in the description of step 54 above, the route length can be indicated as a distance or a time. The user can also specify a one-way route from point A to point B or a loop route starting at point A and finishing at point A. When the user selects a loop route based on a distance selected by the user, the planned loop route might not fit the distance perfectly because the real route cannot be as accurate as an exercise machine. Therefore, the planning for the route preferably allows for a distance error (difference) between the selected distance and the actual distance that the routing software 36 generates. For example, the personal navigation device 10 could notify the user, "The loop route is 5 meters longer than your selection". The user could select "OK" if he accepts. Certainly, the error should be within a predetermined range.

After the user has selected a terrain type or difficulty level and a route length, the routing software 36 consults the map database 32 and the terrain database 34 for generating an exercise route conforming the user's selections. The generated exercise route may be chosen such that unsafe areas indicated in the points of avoidance data 38 are avoided. Otherwise, if the generated exercise route enters areas that are considered to be unsafe, the personal navigation device 10 can notify the user about these unsafe areas.

If the user decides that he likes the planned exercise route that the routing software 36 generated, the user can choose to save the planned exercise route as a favorite exercise route in the memory 30 of the personal navigation device 10. In this way, the user can conveniently revisit this exercise route in the future.

Another feature that the personal navigation device 10 can provide is creating an exercise route with planned stops to allow the user a chance to rest. The user can select the option of "plan a route with stops" when inputting selected parameters for the exercise route to be created. The user can even be offered the choice of selecting the number of stops to be included on the exercise route when the exercise route is planned for a long distance.

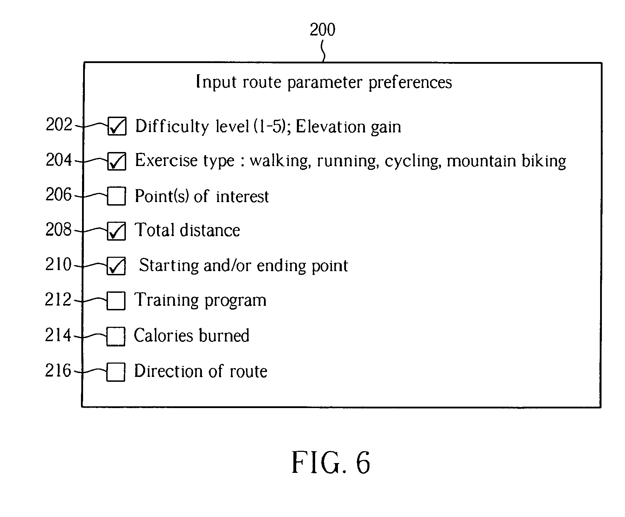

The user can select a variety of input route parameters, each of which is further customizable. Please refer to Fig. 6. Fig. 6 is a screen 200 showing an example of input route parameter preferences selectable by the user. When creating an exercise route, the user may select one or more input route parameter preferences for customizing the type of route the user prefers. In screen 10C, different checkboxes 202, 204, 206, 208, 210, 212, 214, 216 are shown by way of example. Four of the checkboxes 202, 204, 208, 210 have been selected for illustrating which input route parameter preferences are selected by the user when creating a new exercise route. In each case, when one of the input route parameter preferences is selected by the user, the user can be presented with an additional screen corresponding to each individual input route parameter preferences for specifying more detail about the user's input route parameter preferences. A description of each of the input route parameter preferences will be given below.

The difficulty level 202 option can be given a numerical value, such as on a scale of 1 to 5, with 1 being the easiest and 5 being the most difficult. In an embodiment, the difficulty level 202 can be measured by the elevation gain of the exercise route, which can be a net elevation gain (elevation gain minus elevation loss) or can be a gross elevation gain (adding all elevation gains while ignoring elevation loss) . In general, the steeper and the more climbing done on an exercise route, the more difficult the exercise route is as compared to flat exercise routes. It will be appreciated that other factors besides elevation gain, such as terrain, can also be used for determining the difficulty level 202.

The exercise type 204 option presents the user with the chance to select one of several different types of exercise. Different exercise types such as walking, running, cycling, and mountain biking can potentially all be done one different types of terrain. By specifying the exercise type 204, the personal navigation device 10 can limit the search for a suitable exercise route to only those that are appropriate for the chosen exercise type 204.

The point of interest 206 option lets the user request that the created exercise route include one or more points of interest selected by the user. If a large number of points of interest are selected or if a whole category of point of interest in a region are selected, then the created exercise route can try to include as many of the points of interest as possible while at the same time satisfying all of the other input route parameter preferences indicated by the user.

If the user has selected other input route parameters such as distance or starting or ending point, these other selected input route parameters may preclude the routing software 36 from including all points of interest chosen by the user. In this situation, a maximum number of points of interest pertaining to the user's selection will be included. For example, if a user has specified that an exercise route has parameter preferences of a distance of 10 kilometers, a designated starting and ending point, and another parameter preference of seeing as many points of interest within a "Scenic Points" category as possible, the routing software 36 will attempt to fit in as many of the points of interest as possible. If the "Scenic Points" category contains five points of interest in that area, but only three of them can be included in a 10-kilometer route due to the distance constraint, then the routing software 36 will only include those three points of interest in the generated exercise route. On the other hand, when creating an exercise route with no other limiting constraints on the exercise route besides the selected points of interest, then all selected points of interest can be included on the created exercise route.

The total distance 208 option allows the user to limit the created exercise route to one that is approximately a specified distance. The user can also specify any other limitations on the distance, such as less than but not greater than the specified distance, or the distance of the exercise route being within 20% of the specified distance.

The starting and ending point 210 option allows the user to specify one or both of the starting point and the ending point of the exercise route. The starting point can be the same as the ending point of the route or they can be different points. In addition, for instances where the starting point and the ending point are either the same or close to one another, the user can also specify whether there should be any overlap of the departure route and the return route of the exercise route. In other words, the user can specify if any of the route be repeated, or if the departure route and the return route should be different from one another. By choosing a different return route than a departure route, the user can select a complete exercise route having more variety of scenery than if the return route was identical to the departure route. One of the main goals of the present invention is to provide the user with many different options for searching for and creating a customized exercise route so that the exercise route can feel fresh and new to the user, thereby making it more likely that the user will enjoy exercising and continue to use the personal navigation device 10 for creating future exercise routes.

The training program 212 option allows the user to create exercise routes that are consistent with an existing training program that the user is already following and has been entered into the exercise data and preferences 40 portion of memory 30. Based on the results of previous exercise routes that the user has completed, the training program 212 can create an appropriate exercise route for the user on a given day. For example, the length of the created exercise routes can be adjusted according to the user's progress on the training program so that the user is meeting training goals. Using the training program, the user of the personal navigation device 10 receives instructions, suggestions, and motivational encouragement from the personal navigation device 10.

The calories burned 214 option can be used to select an exercise route of an appropriate distance and terrain according to a selected exercise type to ensure that the number of calories that a user burns while exercising on the created exercise route is approximately equal to a specified number. In order to more accurately calculate this value, the user will first have to input various body parameters into the personal navigation device 10 such as the user's height and weight. An exercise route can then be created which aims to enable the user to burn the specified number of calories while exercising.

The direction of route 216 option allows the user to either avoid or use certain geographical directions when the routing software 36 generates the suggested exercise routes. For instance the user may have a reason for selecting or avoiding a certain geographical direction when exercising, such as a strong wind blowing out of a certain direction. If the wind is blowing out of the north, the user may wish to travel on an exercise route that travels only east and west, thereby avoiding travel towards the north. Alternatively, the user may wish to start off the exercise route by traveling north so that the user is heading into the wind while the user still has sufficient energy reserves and then return back to the starting point by traveling south with the wind to the user's back. The user can make these selections using the direction of route 216 option.

The input route parameter preferences shown on the screen 200 in Fig. 6 allow the user to filter down search results when the personal navigation device 10 searches for and creates exercise routes. With every exercise route calculation, alternative routes will be created whenever possible. In this way, the user will be able to select a fresh and appealing exercise route while at the same time selecting an exercise route that conforms to the selected input route parameter preferences.

Besides the input route parameter preferences shown in the screen 200 of Fig. 6, any number of other preferences can be specified by the user such as waypoints to be visited on the exercise route, a direction of a city that the route should head towards, a wind direction to use or avoid, a difficulty level of the exercise route in terms of how flat or steep the exercise route is, heart rate to be achieved by the user on the exercise route, calories to be burned by the user on the exercise route, an expected height or an expected elevation gain of the exercise route, preferred points of interest to be visited on the exercise route, starting and ending points that are different from one other, an unspecified ending point being a specific distance away from the starting point, and surface types such as paved, unpaved, cobblestones, and so on.

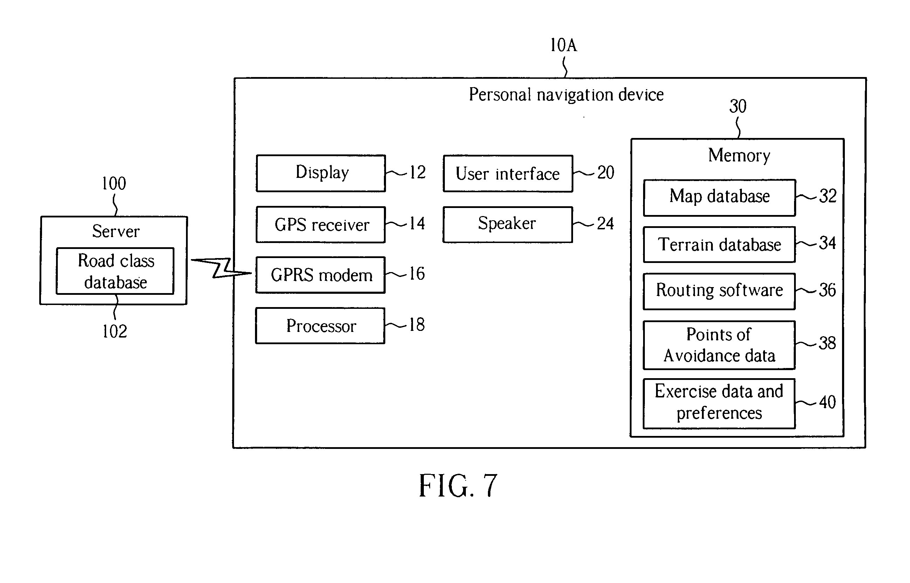

Please refer to Fig. 7. Fig. 7 is a block diagram of a personal navigation device 10A according to another embodiment of the present invention. The personal navigation device 10A contains the GPRS modem 16 for providing internet access. Description of the GPRS modem 16 is not intended to limit the scope of the present invention, and any wireless modem that can connect to the Internet can be used instead of the GPRS modem 16. Other connectivity options besides the GPRS modem that can be used for data transfer include communication according to the ANT+, Wi-Fi, 3G, or Bluetooth protocols.

The GPRS modem 16 allows the personal navigation device 10A to communicate wirelessly with a server 100 via the Internet for accessing a road class database 102 stored in the server 100. Thus, in this embodiment, the map database 32 is stored in the memory 30 of the personal navigation device 10A, whereas the road class database 102 is stored remotely in the server 100. The map database 32 provides information such as street map data, points of interest data, elevation data, and roads suitable for certain modes of transport or exercise such as vehicle, pedestrian, or cycling. The road class database 102 can provide supplemental information as to which roads are specifically suited for types of exercise such as walking, running, cycling, and mountain biking. The user can create routes on the server 100 using the road class database 102, and then the created routes can be sent to the personal navigation device 10A via the GPRS modem 16. The use of the road class database 102 hosted on the server allows a larger amount of data to be used for creating exercise routes, and the road class database 102 can be updated constantly for providing the most up to date road data to the user.

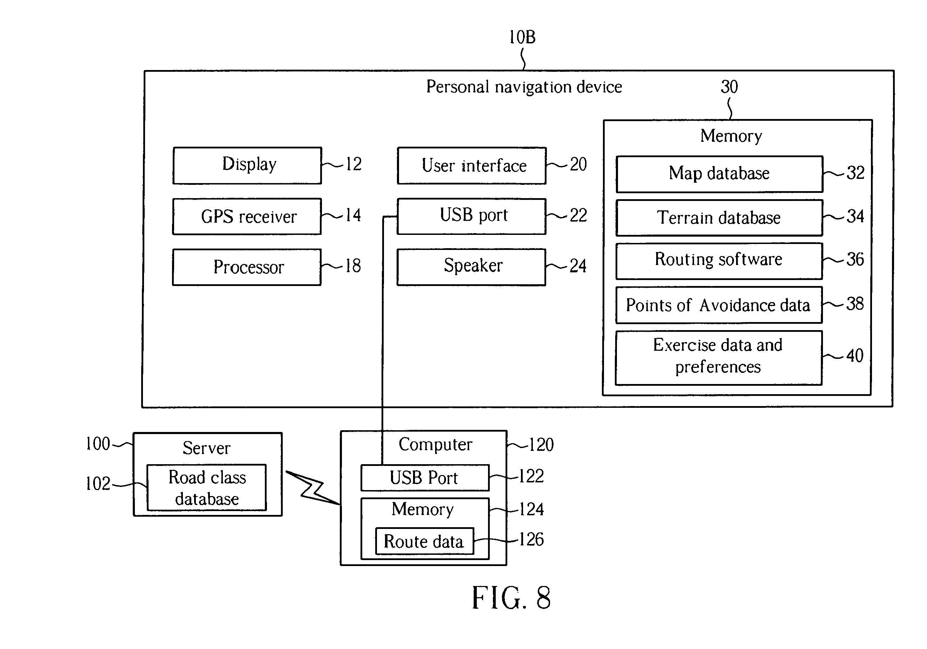

Please refer to Fig. 8. Fig. 8 is a block diagram of a personal navigation device 10B according to another embodiment of the present invention. The personal navigation device 10B contains the USB port 22 for allowing data to be exchanged with a computer 120. The USB port 22 of the personal navigation device 10B communicates with a USB port 122 of the computer 120. The USB port 22 and the USB port 122 are only used as examples, and a variety of other communication port standards can be used instead of USB, such as the Institute of Electrical and Electronics Engineers (IEEE) 1394 standard, the External Serial Advanced Technology Attachment (eSATA) standard, etc. Instead of the personal navigation device 10B directly connecting to the server 100 via the Internet as was shown in Fig. 7, the personal navigation device 10B shown in Fig. 8 connects to the server 100 via the connection to the computer 120. In order to obtain additional exercise route information, the user can first use a route creating application installed on the computer 120 to connect to the road class database 102 stored in the server 100. One or more exercise routes can be created based on the information obtained from the road class database 102, and these exercise routes can be stored as route data 126 in a memory 124 of the computer 120. Then, once the USB port 22 of the personal navigation device 10B is connected to the USB port 122 of the computer 120, the route data 126 can be exported from the computer 120 to the memory 30 of the personal navigation device 10B for use by the routing software 36. Therefore, even if the personal navigation device 10B does not have the capability of connecting directly to the Internet, a communication port such as the USB port 22 can be used to connect to the computer 120, which in turn connects to the Internet for communicating with the server 100.

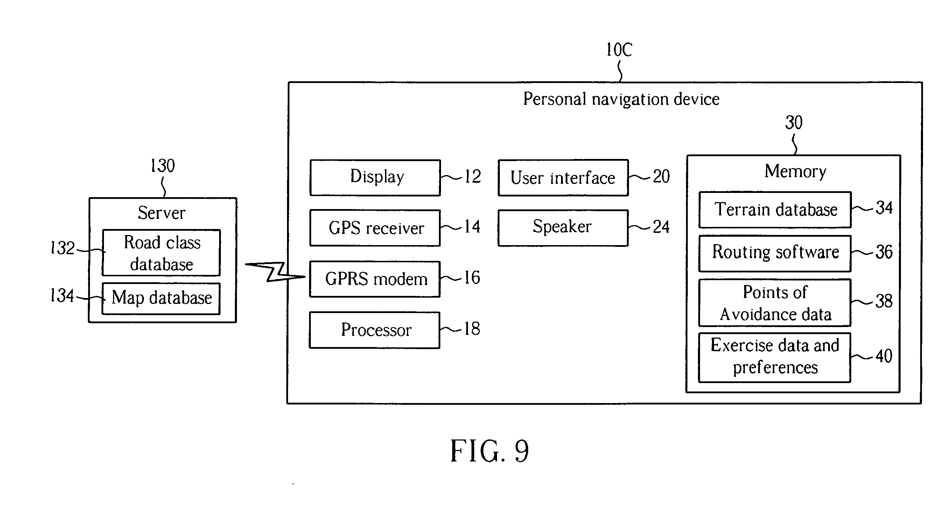

Please refer to Fig. 9. Fig. 9 is a block diagram of a personal navigation device 10C according to another embodiment of the present invention. Like the personal navigation device 10A shown in Fig. 7, the personal navigation device 10C also contains a GPRS modem 16 for communicating with a server 130. Differing from the personal navigation device 10A of Fig. 7, however, the memory 30 of the personal navigation device 10C does not contain a map database, and instead a map database 134 and the road class database 132 are both stored in the server 130. Thus, it is necessary for the personal navigation device 10C to communicate with the server 130 for all database access. An advantage to this system is the required capacity of the memory 30 of the personal navigation device 10C can be considerably reduced. Furthermore, the map database 134 stored in the server 130 can be updated much more frequently than would be feasible for the user of the personal navigation device 10C to update a map database stored locally in the personal navigation device 10C.

A distinguishing feature of the present invention is the use of a "Surprise me" function for surprising the user with automatically generated suggested exercise routes. The user simply needs to indicate the length of the exercise route desired and the routing software 36 of the personal navigation device 10 will automatically generate multiple suggested exercise routes for the user to select from. Once the user selects one of the suggested exercise routes, the routing software 36 prepares navigation instructions for the user to follow the selected exercise route.

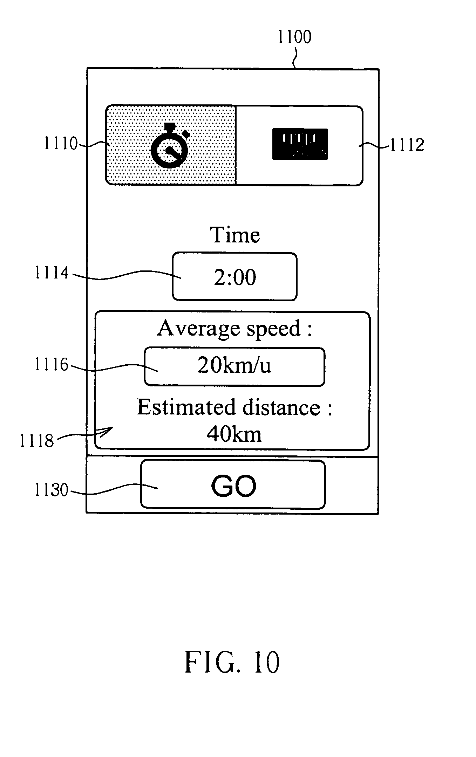

Please refer to Fig. 10. Fig. 10 is a screen 1100 showing settings for creating suggested exercise routes with the surprise me function using a combination of exercise time and average speed at which the user will move while exercising. The screen 1100 shown in Fig. 10 is a simplified example in which only the exercise time and average speed are entered by the user. Other parameters can be specified by the user instead, and the user can specify one or more of time, distance, waypoints to be visited on the exercise route, a direction of a city that the route should head towards, a wind direction to use or avoid, a difficulty level of the exercise route in terms of how flat or steep the exercise route is, heart rate to be achieved by the user on the exercise route, calories to be burned by the user on the exercise route, an expected height or an expected elevation gain of the exercise route, preferred points of interest to be visited on the exercise route, starting and ending points that are different from one other, an unspecified ending point being a specific distance away from the starting point, and surface types such as paved, unpaved, cobblestones, etc. As shown on the screen 1100, the user has selected a time button 1110 for indicating the route length in terms of an entered exercise time 1114 and entered average speed 1116. The entered average speed 1116 can be automatically filled in for the user based on the user's history of average speeds or the user can manually change the entered average speed 1116 to a different value. For instance, the entered exercise time 1114 shown in Fig. 10 is "2:00". Although the time units are not specified on this screen since they are not strictly necessary, the units could also be displayed for the user or even changed by the user. The entered average speed 1116 shown in Fig. 10 is "20km/u", meaning 20 kilometers per unit time. Based on these two entered values, an estimated distance 1118 is calculated to be 40km, meaning that the user would like to generate an exercise route that is 40 kilometers in length.

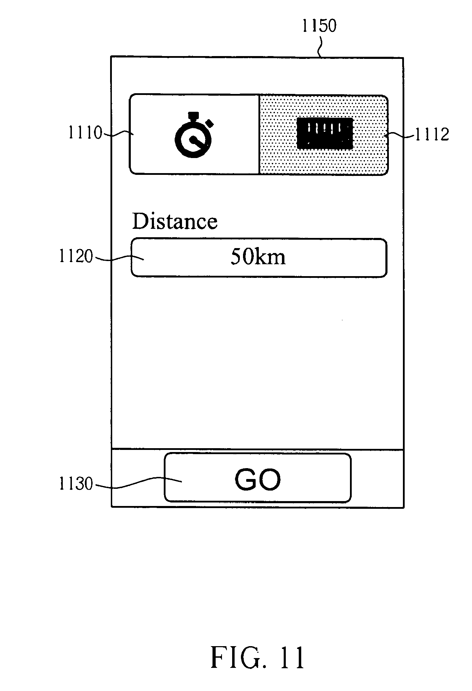

Please refer to Fig. 10 and Fig. 11. Fig. 11 is a screen 1150 showing settings for creating suggested exercise routes with the surprise me function using a distance that the user will travel while exercising. If the user prefers not to input the route length in terms of the entered exercise time 1114 and the entered average speed 1116, the user can instead press a distance button 1112 for indicating the route length in terms of an entered distance 1120. For instance, the entered distance 1120 shown in Fig. 10 is "50km", meaning that the user would like to generate an exercise route that is 50 kilometers in length. After the user has input a combination of the entered exercise time 1114 and the entered average speed 1116 or has input the entered exercise time 1114, the user can press a "GO" button 1130 for indicating that the user has finished indicating the length of the desired exercise route.

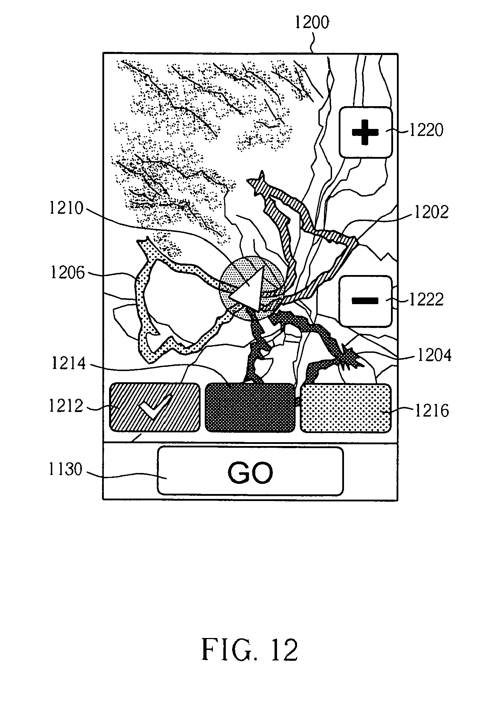

Please refer to Fig. 12. Fig. 12 is a screen 1200 showing a map containing three suggested exercise routes 1202, 1204, 1206 displayed on the map. After the user presses the "GO" button 1130 in either Fig. 10 or Fig. 11, screen 1200 is shown in the user interface 20 of the personal navigation device 10. Each of the three suggested exercise routes 1202, 1204, 1206 is approximately equal to the length of the exercise route that was previously entered by the user. The three suggested exercise routes 1202, 1204, 1206 preferably all have a starting point and an ending point equal or approximately equal to a current position 1210 of the personal navigation device 10, although one-way routes from a starting point to a different ending point could also be used. As shown on screen 1200, the current position 1210 of the personal navigation device 10 is indicated by a triangle. Other indications for the user' s current position 1210 besides the triangle can also be used. Once the user has viewed the three suggested exercise routes 1202, 1204, 1206, the user can select one of the three suggested exercise routes 1202, 1204, 1206 by checking one of the corresponding selection boxes 1212, 1214, 1216. In an embodiment, the selection boxes 1212, 1214, 1216 can also contain the exact distance of the corresponding suggested exercise routes 1202, 1204, 1206. A summary of the suggested exercise routes 1202, 1204, and 1206 can also be provided including height profiles, maximum slopes, average slopes, points of interest on the routes, and so on. For example, as shown in Fig. 12, the selection box 1212 is checked, meaning that the user has selected the suggested exercise route 1202. Once the user has made a selection the user may press the "GO" button 1130 to confirm this selection. If the user does not wish to select any of the three suggested exercise routes 1202, 1204, 1206, the user can simply press a back button on the user interface 20 in order to return to a previous screen such as the screen 1100 or the screen 1150. At that point the "GO" button 1130 can be pressed again to generate another new set of three suggested exercise routes. Other methods can also be used to obtain another set of new suggested exercise routes, including pressing a dedicated button for requesting new exercise routes to be generated. In order to ensure an unlimited number of exercise routes, the present invention strives to present the user with different exercise routes each time the user requests a new set of suggested exercise routes. The present invention will always create different routes so the user can enjoy endless possibilities of exercising.

The user can zoom in or out of the map shown on screen 1200 using a zoom-in button 1220 or a zoom-out button 1222 in order to get a better idea of where the suggested exercise routes 1202, 1204, 1206 lead. Besides zooming, the user can also pan left and right or up and down. If the display 12 is a touch screen then, the zoom and pan functions can be controlled using the touch screen. While screen 1200 shows three suggested exercise routes 1202, 1204, 1206 that have been generated, any number of suggested exercise routes can be generated, including one, two, three, four, or more suggested exercise routes. As long as there are two or more suggested exercise routes, the user will have the ability to select a suggested exercise route that the user prefers from those suggested exercise routes that are displayed at one time.

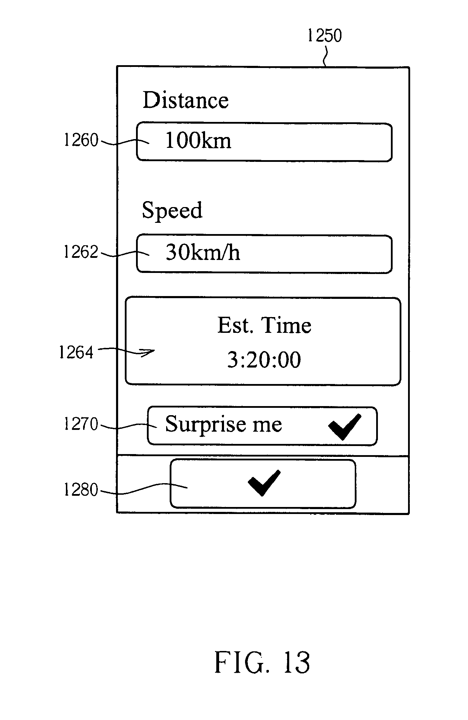

Please refer to Fig. 13. Fig. 13 depicts a workout screen 1250 for allowing the user to enter parameters for a desired workout using a combination of distance that the user will travel while exercising and average speed at which the user will move while exercising. The user can then enter the workout length using an entered distance 1260 and an entered speed 1262. The entered distance 1260 shown in Fig. 13 is "100km", meaning a total length of 100 kilometers. The entered speed 1262 shown in Fig. 13 is "30km/h", meaning the user wishes to travel at an average speed of 30 kilometers per hour. Based on these two entered values, an estimated workout time 1264 is calculated to be "3:20:00", meaning 3 hours and 20 minutes. When entering the workout parameters, the user can toggle a surprise me button 1270 for correspondingly enabling or disabling the surprise me function for automatically generating suggested exercise routes. Once all workout parameters have been entered, the user can confirm this selection by pressing a confirm button 1280. When the user uses the workout screen 1250 to generate a workout while enabling the surprise me function for automatically generating suggested exercise routes, the user can be taken to screen 1200 shown in Fig. 12 upon pressing the confirmbutton 1280 in Fig. 13.

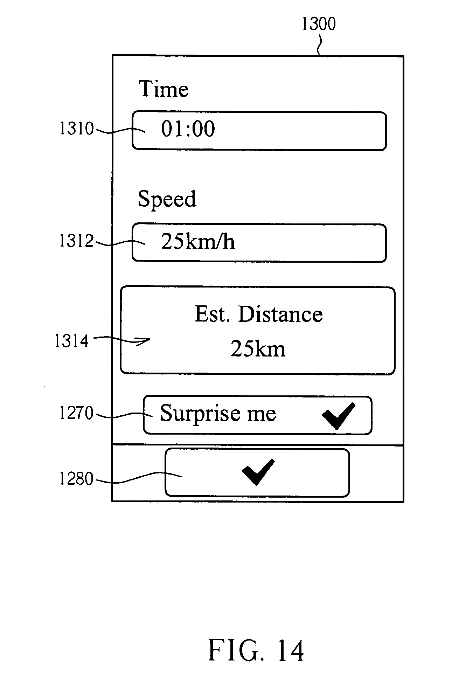

Please refer to Fig. 14. Fig. 14 depicts a workout screen 1300 for allowing the user to enter parameters for a desired workout using a combination of exercise time and average speed at which the user will move while exercising. The user can then enter the workout length using an entered time 1310 and an entered speed 1312. The entered time 1310 shown in Fig. 14 is "01:00", meaning a total exercise time of one hour. The entered speed 1312 shown in Fig. 14 is "25km/h", meaning the user wishes to travel at an average speed of 25 kilometers per hour. Based on these two entered values, an estimated workout distance 1314 is calculated to be "25km", meaning the user will travel over approximately 25 kilometers during the workout. When entering the workout parameters, the user can toggle the surprise me button 1270 for correspondingly enabling or disabling the surprise me function. Once all workout parameters have been entered, the user confirms this selection by pressing the confirm button 1280. When the user uses the workout screen 1300 to generate a workout while enabling the surprise me function for automatically generating suggested exercise routes, the user can be taken to screen 1200 shown in Fig. 12 upon pressing the confirmbutton 1280 in Fig. 14.

Numbers entered by the user in any of the previous screens 1100, 1150, 1250, and 1300 can be remembered by the personal navigation device 10 so that the user does not need to re-enter the exercise time, average exercise speed, or exercise distance every time a new workout is created. The user will still have the ability to modify these values, but if the user does not wish to make any changes the user can quickly confirm the selection since the numbers will be automatically filled in.

In the event of an emergency, the user can perform emergency communication using the GPRS modem 16. The GPRS modem 16 can also be used to communicate with other users, as well as to upload favorite exercise routes via the GPRS modem 16 for sharing the favorite exercise routes on the Internet, and receive training program data from a trainer of the user via the GPRS modem 16.

## Claims

1. A method of creating a customizable exercise route for a user of a personal navigation device (10), the method comprising:
receiving a selection of terrain type or difficulty level from the user;
receiving a selection of a length of an exercise route to be generated; and
**characterized by**:
generating an exercise route for the user according to a current position of the personal navigation device (10), the selected terrain type or difficulty level, and the selected length of the exercise route; and
providing routing instructions for the user to follow the generated exercise route.

2. The method of claim 1, wherein the selected terrain type indicates terrain suitable for walking, running, off-road cycling, or road cycling, and/or
wherein the selected terrain type indicates terrain that is wheelchair accessible, has nearby parking, is in urban areas, is in green belt area, is in natural areas, is beach terrain, is hilly terrain, or is flat terrain, and/or wherein the selected terrain type indicates terrain that includes a predetermined number of stairs, and/or wherein the selected length of the exercise route to be generated is indicated by a period of time the user wishes to exercise or a distance the user wishes to exercise, and/or
wherein the exercise route to be generated is a one-way route or a loop route, wherein preferably when generating a loop route for a selected length, a length of the generated loop route is within a predetermined threshold value of the selected length.

3. The method as in any of claims 1-2 further comprising:
notifying the user about unsafe areas along the generated exercise route, and generating the exercise route comprises avoiding areas considered to be unsafe when generating the exercise route, and/or
further comprising:
saving a favorite suggested exercise route from the first plurality of suggested exercise routes; and
displaying routing instructions for the saved favorite suggested exercise route on a display (12) of the personal navigation device (10) upon receiving selection of the saved favorite suggested exercise route from the user, and/or
further comprising:
receiving a rest stop request from the user; and generating the exercise route for the user including one or more rest stops according to the selected length of the generated exercise route.

4. A method of creating an exercise route for a user, the method comprising:
providing a database comprising geographic data;
receiving a plurality of input route parameters from the user, the input route parameters comprising a type of exercise to be performed on the exercise route; and
**characterized by**:
processing the geographic data from the database and the plurality of input route parameters to define the exercise route from a starting point to an ending point; and
presenting the exercise route to the user.

5. The method of claim 4, wherein the plurality of input route parameters further comprises a selected difficulty level of the exercise route, a total distance to be covered on the exercise route, or a number of calories that the user wishes to burn on the exercise route, and/or
wherein the selected difficulty level of the exercise route indicates a total elevation gain throughout the exercise route, and/or
wherein the type of exercise to be performed on the exercise route is selected from the group consisting of walking, running, cycling, and mountain biking, and/or
wherein the plurality of input route parameters further comprises one or more points of interest to be visited along the exercise route, and/or
wherein the plurality of input route parameters further comprises a category of points of interest to be visited along the exercise route, the exercise route being defined in order to visit a maximum number of points of interest included in the category of points of interest while at the same time satisfying all other input route parameters indicated by the user, and/or
wherein at least one of the starting point and the ending point of the exercise route is selected by the user, wherein preferably the starting point and the ending point of the exercise route are selected to be located at the same place or are selected to be different from each other.

6. The method as in any of claims 4-5, wherein the plurality of input route parameters further comprises current progress on a training program followed by the user, the exercise route being defined to match the user' s training program, and/or
wherein the database comprises a map database and a road class database, the map database comprising street maps, longitude, latitude, and elevation data, and the road class database comprising classifications on roads suitable for various types of exercise, and/or
wherein the exercise route is presented to the user on a personal navigation device (10), the personal navigation device (10) comprising a modem (16) for connecting to both the map database and the road class database via the Internet.

7. A method of creating an exercise route for a user of a personal navigation device (10), the method comprising:
storing a database in a memory (30) of the personal navigation device (10), the database comprising geographic data;
receiving a selection of a length of an exercise route to be generated; and
**characterized by**:
generating a first plurality of suggested exercise routes according to a current position of the personal navigation device (10) and the selected length of the exercise route, wherein each of the first plurality of suggested exercise routes has a starting point and ending point substantially equal to one another and equal to the current position of the personal navigation device (10);
displaying the first plurality of suggested exercise routes to the user on a display (12) of the personal navigation device (10);
receiving, from the user, selection of a selected suggested exercise route from the first plurality of suggested exercise routes; and
displaying routing instructions on the display (12) of the personal navigation device (10) for the selected suggested exercise route.

8. The method of claim 7, wherein the selected length of the exercise route to be generated is indicated by a distance the user wishes to exercise or a period of time the user wishes to exercise along with an estimated speed at which the user will travel while exercising.

9. The method as in any of claims 7-8 further comprising receiving, from the user, selection of a type of exercise to be performed on the exercise route, wherein generating the first plurality of suggested exercise routes comprises generating the first plurality of suggested exercise routes according to the current position of the personal navigation device (10), the selected length of the exercise route, and the selected type of exercise.

10. The method of claim 9, wherein the type of exercise to be performed on the exercise route is selected from the group consisting of walking, running, mountain biking, city biking, and race biking.

11. The method as in any of claims 7-10, wherein after displaying the first plurality of suggested exercise routes to the user on the display (12) of the personal navigation device (10) the method further comprises:
receiving a request from the user to generate a second plurality of suggested exercise routes; and
generating the second plurality of suggested exercise routes according to the current position of the personal navigation device (10) and the selected length of the exercise route in response to receiving the request from the user to generate the second plurality of suggested exercise routes, wherein each of the second plurality of suggested exercise routes has a starting point and ending point substantially equal to one another and equal to the current position of the personal navigation device (10), and the second plurality of suggested exercise routes are different than the first plurality of exercise routes.

12. The method as in any of claims 7-11 further comprising receiving, from the user, selection of a geographical direction to be considered when generating the first plurality of suggested exercise routes.

13. The method of claim 12, wherein the first plurality of suggested exercise routes are created in order to substantially avoid travel in the selected geographical direction, and/or
wherein the first plurality of suggested exercise routes are created in order to substantially avoid travel in the selected geographical direction on beginning or ending legs of the first plurality of suggested exercise routes, and/or
wherein the first plurality of suggested exercise routes are created in order to travel in the selected geographical direction on beginning or ending legs of the first plurality of suggested exercise routes.

14. The method as in any of claims 7-13, further comprising tracking the user's progress on a training program being followed by the user, and generating the first plurality of suggested exercise routes according to the user's progress on the training program, wherein preferably the length of the first plurality of suggested exercise routes is adjusted according to the user's progress on the training program.

15. The method as in any of claims 7-14, further comprising performing emergency communication via a modem (16), and/or
further comprising communicating with users of other personal navigation devices (10) via a modem (16), uploading favorite exercise routes via the modem (16) for sharing the favorite exercise routes on the Internet, and receiving training program data from a trainer of the user via the modem (16).
